# EUROPEAN PATENT APPLICATION

(11) **EP 3 444 273 A1**
(43) Date of publication of application: **20.02.2019**
(21) Application number: 17763615.6
(22) Date of filing: 10.03.2017
(51) Int. Cl.: C07K 16/18, C12N 15/63, G01N 33/574

(54) **ANTIBODY SPECIFICALLY BINDING TO AIMP2-DX2 PROTEIN**

(30) Priority: 10.03.2016 KR 20160028809
(71) Applicant: Medicinal Bioconvergence Research Center, Suwon-si, Gyeonggi-do 16229 (KR); Ewha University-Industry Collaboration Foundation, Seoul 03760 (KR)
(72) Inventor: KIM, Sunghoon, Seoul 06269 (KR); SUNG, Junsik, Seoul 06059 (KR); KWON, Nam Hoon, Seoul 07007 (KR); SHIM, Hyunbo, Seoul 06007 (KR); NGUYEN THI, Thu ha, Seoul 04108 (KR)
(74) Representative: Hoefer & Partner Patentanwälte mbB
(86) International application number: PCT/KR2017/002631
(87) International publication number: WO 2017/155355

(57) **Abstract**

The present invention relates to an antibody specifically binding to AIMP2-DX2 and, more particularly, to an antibody or a fragment of an antibody that specifically binds to AIMP2-DX2 which recognizes a polypeptide comprising the amino acid sequence of SEQ ID NO: 1 as an epitope, a composition for diagnosing cancer comprising the same, and a polynucleotide and recombinant expression vector encoding the antibody, transformed cell using the same and a method for preparing the antibody or fragment of the antibody.

The antibody according to the present invention doesn't bind to wild-type AIMP2, but specifically binds to AIMP2-DX2 mutant protein which has exon2 deletion, it can be usefully used to confirm the presence and expression level of AIMP2-DX2 protein. AIMP2-DX2 protein is associated with pathologies such as cancer and inflammatory diseases and is overexpressed in cancer such as lung cancer, breast cancer, colorectal cancer and the like. Therefore, it can be usefully used to develop a composition for diagnosing the cancer by detecting AIMP2-DX2 as a biomarker for cancer development and progression.

## Description

### Technical Field

The present application claims priority from and the benefit of Korean Patent Application No. 10-2016-0028809 filed on March 10, 2016, which is hereby incorporated by reference for all purposes as if fully set forth herein.

The present invention relates to an antibody specifically binding to AIMP2-DX2 and, more particularly, to an antibody or fragment of the antibody that specifically binds to AIMP2-DX2 which recognizes a polypeptide comprising the amino acid sequence of SEQ ID NO: 1 as an epitope, a composition for diagnosing cancer comprising the same, and a polynucleotide and recombinant expression vector encoding the antibody, a transformed cell using the same and a method for preparing the antibody or fragment of the antibody.

### Background Art

AIMP2(aminoacyl-tRNA synthetase complex-interacting multifunctional protein 2) is one of the proteins involved in the formation of the aminoacyl-tRNA synthetase (ARS) complex. AIMP2 is known to have a function of enhancing TGF-beta signaling through direct interaction with Smad2/3 as a novel tumor suppressor, in addition to its original function in protein synthesis.

As disclosed in Korean Patent No. 10-0762995 by the present inventors, it has been observed that AIMP2-DX2, an exon 2-deficient variant form of AIMP2, is overexpressed in various cancer cell lines and tissues such as lung cancer, liver cancer, skin cancer, breast cancer, kidney cancer, osteosarcoma, etc. Meanwhile, when AIMP2-DX2 is overexpressed by transforming normal cells, the level of wild type (WT) AIMP2 is greatly decreased and the activity of AIMP2 is inhibited such that AIMP2 is prevented from moving to the nucleus, expression of c-myc increased, cell growth was promoted, resulting in abnormal function of TGF-β signal. This shows that AIMP2-DX2 is closely related to cancer development and progression.

In addition, Korean Patent No. 10-1067816 discloses to the public for the first time that AIMP2 mediates apoptotic activity of TNF-alpha through interaction with TRAF2, and that this activity is regulated by AIMP2-DX2. In addition, it has also been discovered that AIMP2-DX2 affects the expression of COX-2, an inflammatory marker, and suggested that the inhibition of AIMP2-DX2 activity may be effective in the treatment of inflammatory diseases.

As AIMP2-DX2 protein has been shown to be involved in the induction and progression of various diseases, AIMP2-DX2 is emerging as a new target substance for new drug development. Therefore, it is necessary to develop a technology for reagents and diagnostic methods that can accurately distinguish and detect mutant AIMP2-DX2 proteins from wild-type AIMP2.

### Detailed Description of the Invention

### Technical Problem

Accordingly, the present inventors screened the Fab phage library for the junction of exon 1 and exon3, which results from exon 2 deletion in the human AIMP2-DX2, thereby completed the present invention by selecting an antibody that doesn't bind to AIMP2 and only specifically binds to AIMP2-DX2.

Therefore, an aspect of the present invention is to provide an antibody or fragment of the antibody that specifically binds to AIMP2-DX2, the antibody or fragment of the antibody recognizing a polypeptide comprising the amino acid sequence of SEQ ID NO: 1 as an epitope.

Another aspect of the present invention is to provide a composition for diagnosing a cancer comprising the antibody or fragment of the antibody.

Still another aspect of the present invention is to provide a polynucleotide encoding the antibody or fragment of the antibody.

Still another aspect of the present invention is to provide a recombinant expression vector comprising the polynucleotide.

Still another aspect of the present invention is to provide a cell transformed with the recombinant expression vector.

Still another aspect of the present invention is to provide a method for preparing an antibody or fragment thereof that specifically binds to AIMP2-DX2, the method comprising the steps of:
(a) transforming a host cell with the recombinant expression vector encoding the antibody or fragment of the antibody;
(b) culturing the host cell and producing the antibody or fragment of the antibody; and
(c) obtaining the antibody or fragment of the antibody produced in the host cell.

Still another aspect of the present invention is to provide use of the antibody or fragment of the antibody for preparing an agent for diagnosing a cancer.

Still another aspect of the present invention is to provide a method for diagnosing a cancer in a subject, the method comprising the steps of:
(a) obtaining a biological sample from the subject;
(b) determining the level of AIMP2-DX2 protein in the biological sample using an antibody or fragment of the antibody; and
(c) comparing the determined level of AIMP2-DX2 protein with the level of AIMP2-DX2 protein in a normal subject.

### Technical Solution

In accordance with an aspect of the present invention, there is provided an antibody or fragment of the antibody that specifically binds to AIMP2-DX2, the antibody or fragment of the antibody recognizing a polypeptide comprising the amino acid sequence of SEQ ID NO: 1 as an epitope.

In accordance with another aspect of the present invention, there is provided a composition for diagnosing a cancer comprising the antibody or fragment of the antibody.

In accordance with another aspect of the present invention, there is provided a polynucleotide encoding the antibody or fragment of the antibody.

In accordance with another aspect of the present invention, there is provided a recombinant expression vector comprising the polynucleotide.

In accordance with another aspect of the present invention, there is provided a cell transformed with the recombinant expression vector.

In accordance with another aspect of the present invention, there is provided a method for preparing an antibody or fragment thereof that specifically binds to AIMP2-DX2, the method comprising the steps of:
(a) transforming a host cell with the recombinant expression vector encoding the antibody or fragment of the antibody;
(b) culturing the host cell and producing the antibody or fragment of the antibody; and
(c) obtaining the antibody or fragment of the antibody produced in the host cell.

In accordance with another aspect of the present invention, there is provided use of the antibody or fragment of the antibody for preparing an agent for diagnosing a cancer.

In accordance with another aspect of the present invention, there is provided a method for diagnosing a cancer in a subject, the method comprising the steps of:
(a) obtaining a biological sample from the subject;
(b) determining the level of AIMP2-DX2 protein in the biological sample using an antibody or fragment of the antibody; and
(c) comparing the determined level of AIMP2-DX2 protein with the level of AIMP2-DX2 protein in a normal subject.

Hereinafter, the present invention will be described in detail.

### The present invention provides an antibody or fragment of the antibody that specifically binds to AIMP2-DX2 which recognizes a polypeptide comprising the amino acid sequence of SEQ ID NO: 1 as an epitope.

In the present invention, 'AIMP2-DX2' means a mutant in which a region of exon 2 in the wild type (WT) AIMP2 protein is deleted. The AIMP2 (aminoacyl-tRNA synthetase complex-interacting multifunctional protein 2) protein is one of the proteins involved in the formation of a complex containing aminoacyl-tRNA synthetases, and is also called p38, JTV-1, JTV1 or p38/JTV-1, and is encoded in the *AIMP2* gene of chromosome 7 (7p22) in humans. The AIMP2 protein is found as a protein consisting of 312 or 320 amino acids (aa), the amino acid sequence of the 312aa protein is disclosed as Genbank accession number AAC50391.1 or GI: 1215669, the amino acid sequence of the 320aa protein is disclosed as Genbank accession number AAH13630.1 or GI: 15489023, etc., and is also described in the literature (312aa version: Nicolaides, N. C., Kinzler, K. W. and Vogelstein, B. Analysis of the 5' region of PMS2 reveals heterogeneous transcripts and a novel overlapping gene, Genomics 29(2), 329-334 (1995); 320aa version: Generation and initial analysis of more than 15,000 full-length human and mouse cDNA sequences, Proc. Natl. Acad. Sci. U.S.A. 99(26), 16899-16903 (2002)).

Thus, in the present invention, the AIMP2-DX2 protein refers to a mutant protein in which the amino acid sequence corresponding to exon2 is deleted from the AIMP2 protein of the 312aa or 320aa. The AIMP2-DX2 protein includes a protein having deletion of the region of exon2 in the entire sequence of AIMP2, and an equivalent of AIMP2 protein (functional equivalent, that is a mutant by substitution, deletion, insertion of amino acid sequences, or a combination of these, having substantially equivalent activity to AIMP2, or functional derivative, that has modification of increasing or decreasing physicochemical properties, having substantially equivalent activity to AIMP2). In addition, the AIMP2-DX2 protein refers to a protein having deletion of total amino acid sequence of exon2 of AIMP2, or having deletion of partial sequence of exon2 so that the amino acid sequnce corresponding to exon1 and exon3 of AIMP2 is located close enough to be recognized by the antibody according to the present invention. Furthermore, it also refers to a protein having deletion of the part of exon1, exon 3, and/or exon4. Preferably, the AIMP2-DX2 protein of the present invention is a protein having the deletion of all exon2 regions of the AIMP2 protein, that is to say 69 amino acids encoded by exon2 is completely deleted that the last amino acid of exon1 of AIMP2 and the first amino acid of exon3 is linked.

In the present invention, 'antibody' is also called immunoglobulin (Ig), and is a generic term of proteins involved in immunity by selectively acting on an antigen. Whole antibodies found in nature generally consist of two pairs of light chain (LC) and heavy chain (HC), polypeptides consisting of several domains, or consist of two pairs of these HC/LC as a basic unit. There are five types of heavy chains that constitute mammalian antibodies: the Greek letters α, δ, ε, γ and µ. Depending on the type of heavy chain, these forms different types of antibodies such as IgA, IgD, IgE, IgG and IgM. There are two types of light chains that constitute mammalian antibodies, of λ and κ.

The heavy and light chains of the antibody are structurally divided into a variable region and a constant region according to the variability of the amino acid sequence. The constant region of the heavy chain consists of three or four heavy chain constant regions such as CH1, CH2 and CH3 (IgA, IgD and IgG antibodies) and CH4 (IgE and IgM antibodies), depending on the type of antibody, and the light chain consists of CL, which is a constant region. The variable region of the heavy chain or the light chain consists of one domain of the heavy chain variable region (VH) or the light chain variable region (VL), respectively. The light and heavy chains are linked by a single disulfide bond, with each variable and constant region aligned side by side, and the two molecules of the heavy chain linked to the light chain is linked via two covalent disulfide bonds to form the entire antibody. The whole antibody binds specifically to the antigen through the variable regions of the heavy chain and the light chain. Since the whole antibody is composed of two heavy and light chain pairs (HC / LC), one molecule of the whole antibody has divalency of single specificity, binding to the same two antigen molecules through two variable region pairs. The antibody variable region binding to the antigen is referred to as an antigen-binding site of the antibody, and the region recognized by the antibody on the surface of the antigen is referred to as an epitope.

A variable region of an antibody comprising an antigen-binding site includes a framework region (FR) having low sequence variability and a complementary determining region (CDR), that is hypervariable region having high sequence variability. Each of VH and VL consists of three CDRs and four FRs, which are arranged from N-terminus to C-terminus in the order of FR1-CDR1-FR2-CDR2-FR3-CDR3-FR4. In the variable region of the antibody, CDRs have the highest sequence variability, directly bind to the antigen, and are most important for the antigen specificity of the antibody.

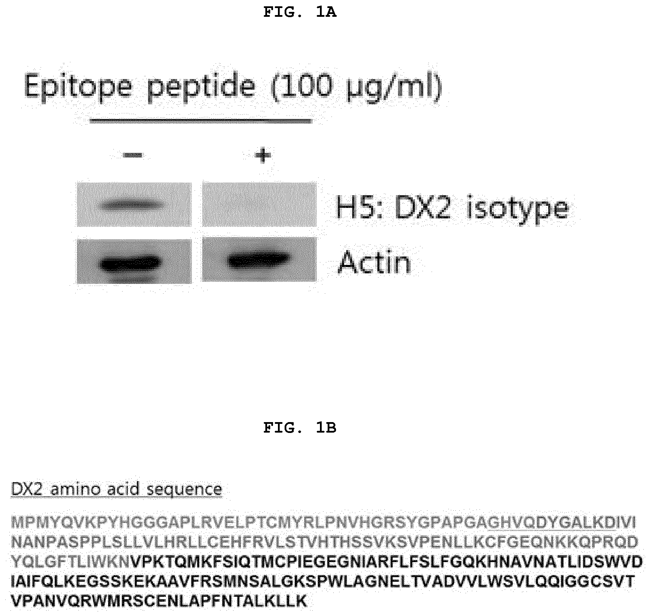

The antibody according to the present invention is an antibody or a fragment of an antibody that specifically binds to AIMP2-DX2 mutant protein, and characteristically recognizes a polypeptide comprising the amino acid sequence of SEQ ID NO: 1 as an epitope. The amino acid sequence of SEQ ID NO: 1 (hereinafter referred to as AIMP2-DX2 antigen peptide) is a sequence of 11 amino acids at the region where exon1 and exon3 are joined in the AIMP2-DX2 protein in which exon2 is completely deleted from the AIMP2 protein, and the amino acid sequence is GHVQDYGALKD. The N-terminal sequence GHVQ corresponds to exon1 and the C-terminal sequence DYGALKD corresponds to exon3 (see Fig. 1B). Since the amino acid sequence of SEQ ID NO: 1 is not present in the wild-type AIMP2 protein without exon2 deletion, the antibody or fragment of the antibody recognizing SEQ ID NO: 1 as the epitope does not bind to the wild-type AIMP2 protein, but only specifically binds to the AIMP2-DX2 mutant protein.

The present inventors screened a phage-displayed Fab library in order to develop an antibody recognizing the amino acid sequence of SEQ ID NO: 1 as an epitope, and specifically binding to the AIMP2-DX2 mutant protein. Briefly, a cysteine (Cys) amino acid for conjugating a carrier protein to a polypeptide having the amino acid sequence of SEQ ID NO: 1 was added to the C-terminus and conjugated the carrier protein, keyhole limpet hemocyanin, and the carrier protein-conjugated peptide was injected to rabbits to elicit immune response. The cDNA corresponding to the variable regions of the rabbit antibody was synthesized from the mRNA extracted from the spleen of the immune response-induced rabbits and assembled with the DNA encoding the antibody constant regions of the human Fab, and inserted into the pComb3XTT vector used for phage display. The Fab library prepared by transforming the vector encoding the rabbit / human chimera Fab into Escherichia coli (ER2537) and infecting with a helper phage was panned against the AIMP2-DX2 protein, and clones that bind to AIMP2-DX2 protein were selected.

The present inventors have conducted experiments such as antigen pre-adsorption and inhibition of antigen expression using siRNA, and found that the H5 antibody and its fragment (Fab) selected from the Fab library screening specifically bind to the AIMP2-DX2 mutant protein. The binding between the H5 antibody and the AIMP2-DX2 variant is not affected by the N-terminal amino acid deletion that affects the solubility of the AIMP2-DX2 variant protein. In particular, the antibody according to the present invention is highly effective in selectively detecting the AIMP2-DX2 mutant protein by binding only to the AIMP2-DX2 protein without binding to the wild-type AIMP2 protein at all.

The antibody or fragment of the antibody according to the present invention can be preferably characterized by comprising:
a heavy chain variable region comprising a heavy chain complementary-determining region (CDR) 1 comprising the amino acid sequence of SEQ ID NO: 3, a heavy chain CDR2 comprising the amino acid sequence of SEQ ID NO: 5 and a heavy chain CDR3 comprising the amino acid sequence of SEQ ID NO: 7; and a light chain variable region comprising a light chain CDR1 comprising the amino acid sequence of SEQ ID NO: 9, a light chain CDR2 comprising the amino acid sequence of SEQ ID NO: 11 and a light chain CDR3 comprising the amino acid sequence of SEQ ID NO: 13.

In addition, the antibody or fragment of the antibody according to the present invention can be preferably characterized by consisting of:
a heavy chain variable region comprising a heavy chain complementary-determining region (CDR) 1 comprising the amino acid sequence of SEQ ID NO: 3, a heavy chain CDR2 comprising the amino acid sequence of SEQ ID NO: 5 and a heavy chain CDR3 comprising the amino acid sequence of SEQ ID NO: 7; and a light chain variable region comprising a light chain CDR1 comprising the amino acid sequence of SEQ ID NO: 9, a light chain CDR2 comprising the amino acid sequence of SEQ ID NO: 11 and a light chain CDR3 comprising the amino acid sequence of SEQ ID NO: 13.

In addition, the antibody or fragment of the antibody according to the present invention can be preferably characterized by essentially consisting of:
a heavy chain variable region comprising a heavy chain complementary-determining region (CDR) 1 comprising the amino acid sequence of SEQ ID NO: 3, a heavy chain CDR2 comprising the amino acid sequence of SEQ ID NO: 5 and a heavy chain CDR3 comprising the amino acid sequence of SEQ ID NO: 7; and a light chain variable region comprising a light chain CDR1 comprising the amino acid sequence of SEQ ID NO: 9, a light chain CDR2 comprising the amino acid sequence of SEQ ID NO: 11 and a light chain CDR3 comprising the amino acid sequence of SEQ ID NO: 13.

In addition, the antibody or fragment of the antibody according to the present invention can be characterized by comprising the CDRs of the heavy and light chain, and comprising a heavy chain variable region(VH) comprising the amino acid sequence of SEQ ID NO: 15; and a light chain variable region(VL) comprising the amino acid sequence of SEQ ID NO: 17.

In addition, the antibody or fragment of the antibody according to the present invention can be characterized by comprising the CDRs of the heavy and light chain, and consisting of a heavy chain variable region(VH) comprising the amino acid sequence of SEQ ID NO: 15; and a light chain variable region(VL) comprising the amino acid sequence of SEQ ID NO: 17.

In addition, the antibody or fragment of the antibody according to the present invention can be characterized by comprising the CDRs of the heavy and light chain, and essentially consisting of a heavy chain variable region(VH) comprising the amino acid sequence of SEQ ID NO: 15; and a light chain variable region (VL) comprising the amino acid sequence of SEQ ID NO: 17.

The antibody according to the present invention can be characterized by comprising a heavy chain comprising the amino acid sequence of SEQ ID NO: 19; and a light chain comprising the amino acid sequence of SEQ ID NO: 21.

In addition, the antibody according to the present invention can be characterized by consisting of a heavy chain comprising the amino acid sequence of SEQ ID NO: 19; and a light chain comprising the amino acid sequence of SEQ ID NO: 21.

In addition, the antibody according to the present invention can be characterized by essentially consisting of a heavy chain comprising the amino acid sequence of SEQ ID NO: 19; and a light chain comprising the amino acid sequence of SEQ ID NO: 21.

The antibody or fragment of the antibody according to the present invention is not limited as long as it has the CDR, VH and VL, or the light chain and the heavy chain structure, and the antibody can be an antibody of IgG, IgA, IgM, IgE or IgD form. Preferably it can be an IgG form of the antibody.

In addition, the fragment of the antibody according to the present invention refers to the fragment of the antibody that retains binding specificity to the AIMP2-DX2 antigen, specifically it can be a form of Fab, F(ab)₂, Fab', F(ab')₂, Fv, diabody, scFv, etc. Fab (fragment antigen-binding) is an antigen-binding fragment of an antibody, consisting of one variable region and one constant region of each of the heavy and light chains. F(ab')₂ is a fragment produced by hydrolyzing an antibody using pepsin, and has a form of two Fabs linked by a disulfide bond at heavy chain hinge. F(ab') is a monomer antibody fragment in which a heavy chain hinge is added to a Fab obtained by reducing disulfide bonds of F(ab')₂ fragment. Fv(variable fragment) is an antibody fragment consisting of only variable regions of heavy and light chains, respectively. A single chain variable fragment (scFv) is a recombinant antibody fragment in which VH and VL are linked by a flexible peptide linker. A diabody is a form of a fragment in which VH and VL of a scFv are linked by a very short linker and cannot bind to each other but form a dimer by binding with VL and VH of another scFv of the same type, respectively.

In addition, the antibody according to the present invention can be a monoclonal antibody derived from a single B cell or a polyclonal antibody derived from a plural B cells, and preferably it is a monoclonal antibody that is a group of an antibody having substantially the same amino acid sequence of heavy and light chains of the antibody. The antibody or fragment of the antibody according to the present invention can be derived from any animal, including mammals, birds, etc., including humans, and can be a chimera antibody or a fragment of the antibody having antibody sequence of different species. The antibody or fragment thereof of the present invention may be conjugated with an enzyme, a fluorescent substance, a radioactive substance and a protein etc, but is not limited thereto.

### The present invention also provides a composition for diagnosing a cancer comprising the antibody or fragment of the antibody according to the present invention.

The AIMP2-DX2 mutant protein is overexpressed in a variety of cancers and is known to be closely related to the development and progression of cancer. AIMP2-DX2 is specifically overexpressed in various types of cancer cell lines and cancer tissues such as lung cancer, liver cancer, skin cancer, breast cancer, kidney cancer, osteosarcoma, etc. according to the present inventors(Korean patent No.10-0762995). When AIMP2-DX2 is overexpressed, the level of wild-type AIMP2, which is a tumor suppressor function, is dramatically decreased and its activity such as moving to nucleus is inhibited. In addition, AIMP2-DX2 overexpression increases the expression of c-myc, blocks the migration of AIMP2 to the nucleus, causes abnormalities of TGF-β signaling, and there is a close relationship between the level of AIMP2-DX2 expression and development and progression of cancer. Thus, it can be known that cancer can be diagnosed by detecting abnormal expression or abnormal overexpression of AIMP2-DX2.

The composition for diagnosing a cancer according to the present invention can diagnose without limitation as long as it is a cancer that abnormally expresses or overexpresses AIMP2-DX2. For example, colorectal cancer, colon cancer, lung cancer, liver cancer, gastric cancer, esophageal cancer, pancreatic cancer, gallbladder cancer, kidney cancer, bladder cancer, prostate cancer, testicular cancer, cervical cancer, endometrial cancer, villous cancer, ovarian cancer, breast cancer, thyroid cancer, brain cancer, head and neck cancer, melanoma, lymphoma, aplastic anemia and blood cancer etc., can be diagnosed, preferably, the composition can be used for diagnosing breast cancer, colon cancer, colorectal cancer, lung cancer, liver cancer, skin cancer, kidney cancer, osteosarcoma, etc. In addition to the antibody or fragment of the antibody according to the present invention, the composition for diagnosing a cancer according to the present invention can further comprise elements commonly used for detecting proteins with antibodies such as secondary antibodies, substrates for colorimetric reaction of secondary antibodies, cofactors, etc.

### The present invention also provides a polynucleotides encoding the antibody or fragment of the antibody according to the present invention.

Specifically, the polynucleotide can be a polynucleotide comprising the nucleotide sequence of SEQ ID NO.16 encoding a heavy chain; or nucleotide sequence of SEQ ID NO.18 encoding a light chain of the antibody according to the present invention.

### The present invention also provides a recombinant expression vector comprising the polynucleotide encoding the antibody or fragment of the antibody according to the present invention.

In the present invention, 'recombinant' can be used in a manner compatible with 'genetic manipulation' and refers to preparing a gene that does not exist in the natural state using the experimental technique of molecular cloning such as transforming, cutting or linking genes. In the present invention, 'expression' means that a protein or a nucleic acid is produced in a cell.

In the present invention, the 'recombinant expression vector' is a vector capable of expressing a desired protein or nucleic acid (RNA) in a suitable host cell, and is a gene construct comprising essential regulating elements which are operably linked to polynucleotide(gene) insert for its expression. The term 'operably linked' refers to a 'functional linkage' between a nucleic acid expression control sequence and a nucleic acid sequence encoding a desired protein or RNA to perform a general function, and the gene can be expressed by the expression control sequence. The 'expression control sequence' refers to a DNA sequence that is operably linked to and controls the expression of a polynucleotide sequence in a specific host cell. Such control sequences include, but are not limited to, promoters for conducting transcription, any operator sequences for regulating transcription, sequences encoding suitable mRNA ribosome binding sites, sequences regulating termination of transcription and translation, initiation codons, termination codons, polyadenylation signal and enhancer etc.

The recombinant expression vector of the present invention is not particularly limited as long as it is a vector conventionally used in the field of cloning and antibody production. Examples of the recombinant expression vector include, but are not limited to, a plasmid vector, a cosmid vector, a bacteriophage vector, a viral vector, etc. The plasmids derived from Escherichia coli (pBR322, pBR325, pUC118 and pUC119, pET-22b (+)), plasmids derived from Bacillus subtilis (pUB110 and pTP5) and plasmids derived from yeast (YEp13, YEp24 and YCp50) can be used as the plasmid, and animal viruses such as retrovirus, adenovirus or vaccinia virus, insect viruses such as baculovirus, and the like can be used as the virus. Vectors of the pComb3 family commonly used in phage display can be used, and in order to express the antibody in mammalian cells, vectors commonly used for expressing proteins in mammalian cells such as pcDNA or pVITRO can be used.

### The present invention also provides a cell transformed with the recombinant expression vector comprising a polynucleotide encoding the antibody or fragment of the antibody according to the present invention.

The cell of the present invention is not particularly limited as long as it is a cell that can be used for expressing a polynucleotide encoding the antibody or the fragment thereof comprised in the recombinant expression vector of the present invention. The cells (host cells) transformed with a recombinant expression vector according to the present invention can be prokaryotic (e.g., E. coli), eukaryotes (e.g. yeast or other fungi), plant cells (e.g., tobacco or tomato plant cell), animal cell (e.g., a human cell, a monkey cell, a hamster cell, a rat cell, a mouse cell), insect cells or hybridoma derived from those cells. For example, HEK293T or 293F cell can be used.

Recombinant expression vectors capable of expressing polypeptides of the antibody or fragment of the antibody according to the present invention can transform a cell by being introduced into a cell for producing the antibody or fragment of the antibody, by methods known in the art such as, but not limited to, transient transfection, microinjection, transduction, cell fusion, calcium phosphate precipitation, liposome-mediated transfection, DEAE dextran-mediated transfection, polybrene-mediated transfection, electroporation, gene gun, and known methods for introducing a nucleic acid into a cell. The cells transformed with the recombinant expression vector according to the present invention produce a heavy chain, light chain or fragment of the antibody according to the present invention.

### The present invention also provides a method for preparing an antibody or fragment thereof that specifically binds to AIMP2-DX2, the method comprising the steps of: (a) transforming a host cell with the recombinant expression vector comprising the polynucleotide encoding the antibody or fragment of the antibody according to the present invention; (b) culturing the host cell and producing the antibody or fragment thereof; and (c) obtaining the antibody or fragment thereof produced in the host cell.

(a) is a step of transforming a host cell for producing an antibody or fragment thereof according to the present invention with a recombinant expression vector in which a polynucleotide encoding the antibody or fragment thereof is operably linked.

A person skilled in the art can perform this step by selecting an appropriate transformation method as described above according to the type of recombinant expression vector and the host cell selected. The recombinant expression vector comprising the base sequences of a heavy chain and a light chain can be co-transformed into the same host cell so that the heavy chain and the light chain are expressed in a single cell, or the recombinant expression vector comprising the base sequence of the heavy chain and the light chain can be transformed into separate cells so that the heavy chain and the light chain are separately expressed.
(b) is a step of culturing the transformed host cell to produce a polypeptide of the heavy chain, light chain or antibody fragment of the antibody of the present invention from the recombinant expression vector introduced into the host cell.

A person skilled in the art can appropriately select medium composition, culture conditions, culture time, etc., for culturing the selected host cells. Antibody molecules produced in the host cell can be accumulated in the cytoplasm of the cell, or can be secreted outside the cell or in the culture medium by a suitable signal sequence, or targeted to the periplasm or the like. In addition, it is preferred that the antibody according to the present invention is refolded and has a functional conformation by using methods known in the art to maintain binding specificity for AIMP2-DX2. In the case of producing the antibody of the IgG type, the heavy chain and the light chain can be expressed in separate cells and the heavy chain and the light chain can be contacted in separate steps to form the complete antibody, or the heavy chain and the light chain can be expressed in the same cell to form the complete antibody inside the cell.
(c) is a step of obtaining the antibody or fragment thereof produced in the host cell.

A person skilled in the art can appropriately select and control the method of obtaining the antibody or fragment thereof taking into account the characteristics of the antibody or fragment polypeptide thereof produced in the host cell, the characteristics of the host cell, the mode of expression or the targeting of the polypeptide. For example, the antibody or fragment thereof secreted in the culture medium can be recovered by a method such as obtaining the medium in which the host cell has been cultured, removing the impurities by centrifugation, or the like. If necessary, the cells may be dissolved in an extent that does not affect the functional structure of the antibody or fragment thereof in order to release and recover the antibody present in the specific organelles or cytoplasm in the cell to the outside of the cell. Further, the obtained antibody can be further subjected to a process of further removing the impurities and concentrating through a method such as chromatography, filtration by a filter or the like or dialysis, etc.

The present invention provides use of the antibody or fragment thereof for preparing an agent for diagnosing a cancer.

The present invention provides a method for diagnosing a cancer in a subject, the method comprising the steps of: (a) obtaining a biological sample from the subject; (b) determining the level of AIMP2-DX2 protein in the biological sample using the antibody or fragment thereof of any one of claims 1 to 6; and (c) comparing the determined level of AIMP2-DX2 protein with the level of AIMP2-DX2 protein in a normal subject.

The "biological sample" or "sample" includes blood and other liquid samples having biological origins, biopsy samples, solid tissue samples such as tissue culture, or cells derived therefrom. More specifically, examples of the biological sample may include, but are not limited to, tissues, extracts, cell lysates, whole blood, plasma, serum, saliva, ocular fluid, cerebrospinal fluid, sweat, urine, milk, ascites fluid, synovial fluid, peritoneal fluid, and the like. The sample may be obtained from animals, preferably mammals, and most preferably humans. The sample may be pre-treated before its use for detection. For example, the sample may be pre-treated by filtration, distillation, extraction, concentration, inactivation of interference components, reagent addition, and the like. In addition, nucleic acid and proteins isolated from the sample may be used for detection.

The 'subject' of the present invention can be an animal, preferably a mammal, particularly an animal including a human, and can be an animal derived cell, tissue, organs and the like. The subject can be a patient requiring the effect.

The diagnostic method of the present invention can diagnose the actual disease occurrence by comparing the AIMP2-DX2 protein level in the biological sample of the normal subject with the protein level in the biological sample of the cancer-suspected subject. That is, the level of AIMP2-DX2 protein is measured using the antibody or fragment thereof of the present invention from a biological sample of the subject suspected of having cancer, and the level of AIMP2-DX2 protein is measured from the biological sample of the normal subject using the antibody or fragment thereof of the present invention, and after comparing the two, in the case AIMP2-DX2 protein level of the suspected subject is abnormally expressed or overexpressed compare to the normal subject, it can be diagnosed as a corresponding disease. Therefore, the diagnostic method can further comprises the step of (d) determining that the cancer has developed when the expression level of the AIMP2-DX2 protein is abnormally expressed or overexpressed compared to the normal subject. In this regard, the level of AIMP2-DX2 in body fluids is based on what has been reported to be increased in various cancer cell lines and tissues, particularly lung cancer, liver cancer, skin cancer, breast cancer, kidney cancer, osteosarcoma, and the like. The cancer is as described above.

The diagnostic method is accomplished through a specific antigen-antibody interaction between the AIMP2-DX2 protein and the antibody or fragment thereof of the present invention, and the amount of the antigen-antibody complex produced by the reaction can be quantitatively determined through the strength of the signal of detection label(detection label). The term 'antigen-antibody complex' in the present invention refers to a complex of the AIMP2-DX2 protein and the antibody or fragment thereof of the present invention which is specific to AIMP2-DX2 protein.

The detection label can be selected from the group consisting of enzymes, fluorescent materials, ligands, luminescent materials, microparticles, redox molecules, and radioisotopes, but are not limited thereto. When the enzyme is used as the detection label, examples of the usable enzyme may be β-glucuronidase, β-D-glucosidase, β-D-galactosidase, urease, peroxidase or alkaline phosphatase, acetylcholine esterase, glucose oxidase, hexokinase and GDPase, RNase, glucose oxidase, luciferase, phospho-fructokinase, phosphoenolpyruvate, carboxylase, aspartate amino transferase, phosphenol pyruvate decarboxylase, β-lamatase, or the like, but are not limited thereto. Examples of the fluorescent material may be fluorescein, isothiocyanate, rhodamine, phycoerythrin, phycocyanin, allophycocyanin, o-phthaldehyde, fluorescamin, or the like, but are not limited thereto. Examples of the ligand may be biotin derivatives or the like, but not limited thereto. Examples of the luminescent material may be acridinium ester, luciferin, luciferase, or the like, but are not limited thereto. Examples of the microparticles may be colloidal gold, colored latex, or the like, but are not limited thereto. Examples of the redox molecule may be ferrocene, ruthenium complexes, viologen, quinone, Ti ions, Cs ions, diimide, 1,4-benzoquinone, hydroquinone, K₄W(CN)₈, [Os(bpy)₃]²⁺, [RU(bpy)₃]²⁺, [MO(CN)₈]⁴, or the like, but are not limited thereto. Examples of the radioisotope may be ³H, ¹⁴C, ³²P, ³⁵S, ³⁶Cl, ⁵¹Cr, ⁵⁷Co, ⁵⁸Co, ⁵⁹Fe, ⁹⁰Y, ¹²⁵I, ¹³¹I, ¹⁸⁶Re, or the like, but are not limited thereto.

Analytical methods for measuring protein levels include Western blotting, ELISA, radioimmunoassay, radial immunodiffusion, Ouchterlony immunodiffusion, rocket immunoelectrophoresis, immunohistochemistry, immunoprecipitation assays, complement fixation assays, FACS, protein chips, and the like, but not limited thereto. Through these analytical methods, it is possible to compare the amount of the antigen-antibody complex formed in the normal control group with the amount of the antigen-antibody complex formed in the suspected cancer patient, and it is possible to diagnose the actual cancer incidence in a suspected disease patient by determining the abnormal expression or overexpression of AIMP2-DX2 such as increase in production level.

The term "comprising" is used synonymously with "containing" or "being characterized", and does not exclude additional ingredients or steps that are not mentioned in the compositions and the methods. The term "consisting of" excludes additional elements, steps, or ingredients that are not separately described. The term "essentially consisting of" means that in the scope of the compositions or methods, the term includes described materials or steps as well as any material or step that does not substantially affect basic characteristics of the compositions or methods.

### Advantageous Effects

Accordingly, the present invention provides an antibody or fragment thereof specifically binding to AIMP2-DX2 protein, a composition for diagnosing a cancer comprising the same, and a polynucleotide encoding the antibody, a recombinant expression vector, a cell transformed with the same, a method for preparing the antibody or fragment of the antibody. Since the antibody and fragment of the antibody provided in the present invention specifically bind to AIMP2-DX2 mutant protein, which has exon2 deletion, without reacting with wild-type AIMP2, it is effective in determining the presence or expression level of AIMP2-DX2 protein which is related to various pathologies such as cancer and inflammatory diseases, and the like.

### Brief Description of the Drawings

FIG.1 shows an experiment to confirm the binding specificity of H5 Fab to the AIMP2-DX2 antigen peptide. FIG. 1A shows the result of Western blot detecting the H460 cell (a lung cancer cell line) lysate using H5 Fab with antigen peptide (epitope peptide, 100µg/ml, indicated as "+"), or without antigen peptide (indicated as "-"). FIG. 1B shows a portion corresponding to the antigen peptide used for screening the H5 antibody in the amino acid sequence of AIMP2-DX2 in which exon2 is deleted in wild-type AIMP2 (underlined portion, amino acid sequence GHVQDYGALKD). exon1, exon3, and exon4 are displayed in gray, green, and black, respectively.

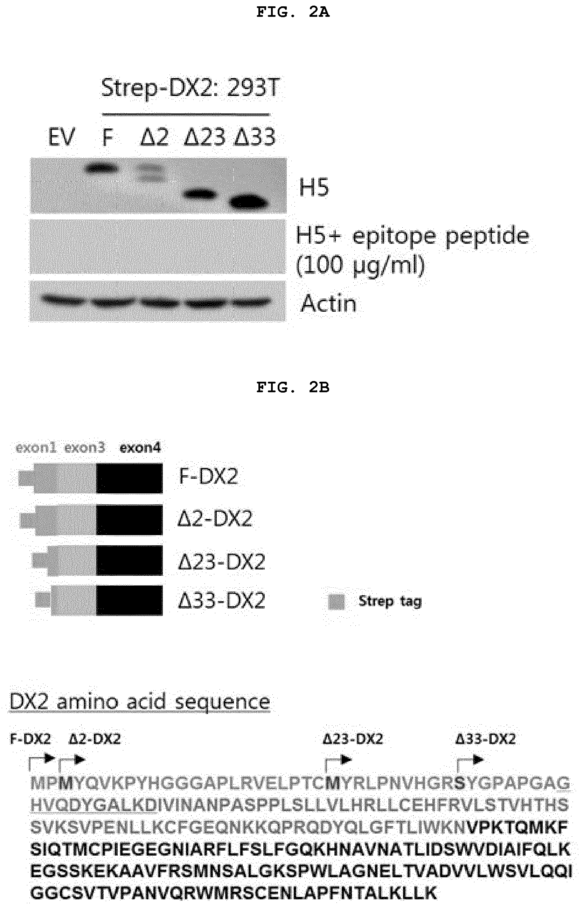

FIG.2 shows an experiment to confirm the binding specificity of the H5 Fab to the AIMP2-DX2 antigen. FIG. 2A shows the results of western blot using H5 Fab and H460 cell lysate, in which H460 cell was transformed with AIMP2-DX2 siRNA(siDX2, "DX2"; si exon4 #1, "#1"; si exon4 #2, "#2") and cultured for 72 hours. FIG. 2B shows the target site of the siRNA used in the experiment. siDX2 targets the exon1-exon3 junction of AIMP2-DX2 and si exon4 #1 and #2 target different regions of exon4, respectively. "c" means control.

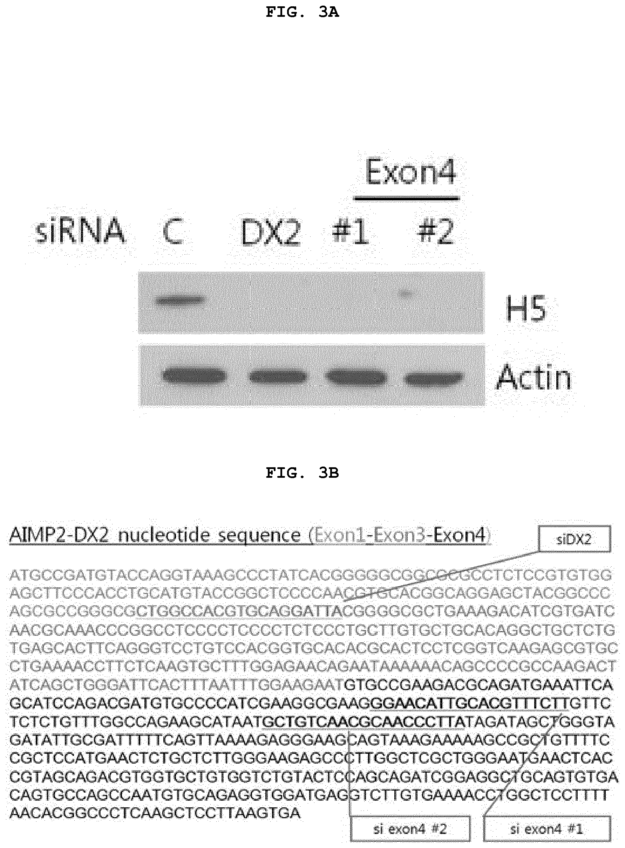

FIG.3 shows an experiment for confirming the binding of H5 Fab to the N-terminal deleted AIMP2-DX2 protein. FIG. 3A shows the result of Western blot detecting the HEK293T cell lysate using H5 Fab with antigen peptide (epitope peptide, 100µg/ml, "H5+epitope peptide"), or without antigen peptide ("H5"), in which the HEK293T cell was transformed to express various forms of AIMP2-DX2 protein. The AIMP2-DX2 protein expressed in HEK293T cells is conjugated with strep tag, and is full-length protein(F-DX2, "F"), or the protein lacking N-terminal amino acids ("Δ2", lacking 2 amino acids, Δ2-DX2; "Δ23", lacking 23 amino acids, Δ23-DX2; "Δ33", lacking 33 amino acids, Δ33-DX2). This result shows that H5 Fab recognizes various forms of AIMP2-DX2 containing the epitope. "EV" means an empty vector. FIG. 3B shows a schematic diagram of the AIMP2-DX2 proteins above (exon 1, exon 3 and exon 4 are represented by gray, green and black, respectively, strep tag is represented by orange color and composed of 30 amino acids including linker) and regions corresponding to the AIMP2-DX2 amino acid sequence (amino acids indicated with black arrow and red color).

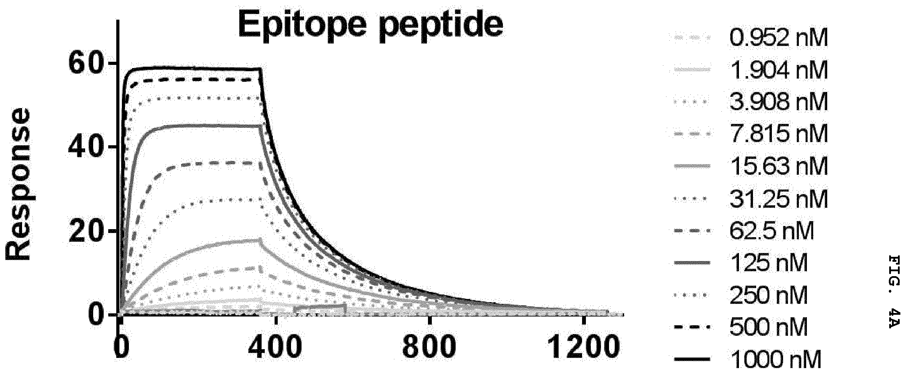

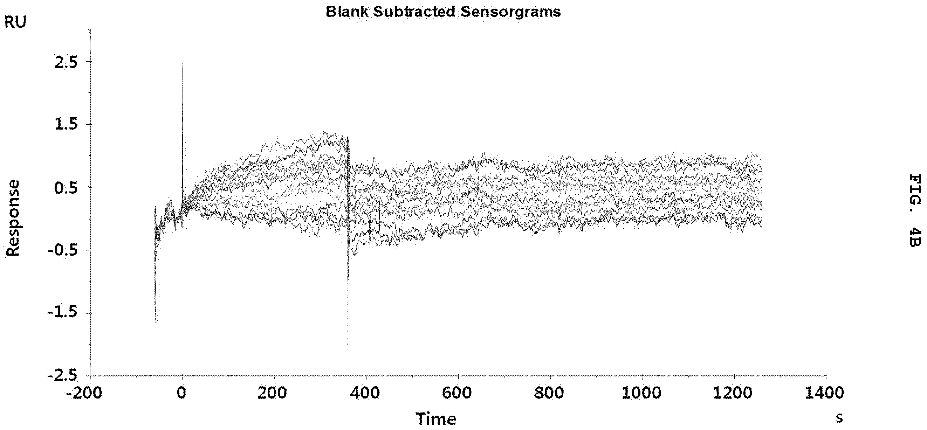

FIG. 4 shows a surface plasmon resonance experiment for confirming the binding affinity between the H5 IgG antibody and the AIMP2-DX2 antigen peptide. The H5 antibody and the IgG protein were coated on a CM5 chip, and the AIMP2-DX2 antigen peptide was flowed at various concentrations, and then the degree of the binding reaction between the antigen peptide and the antibody was shown as sensorgrams. The x-axis of the sensorgram represents the time (seconds, s), and the y-axis represents the response unit (RU). The box shows the concentration of the AIMP2-DX2 antigen peptide (all in nM) and is the same in both A and B of FIG. 4.

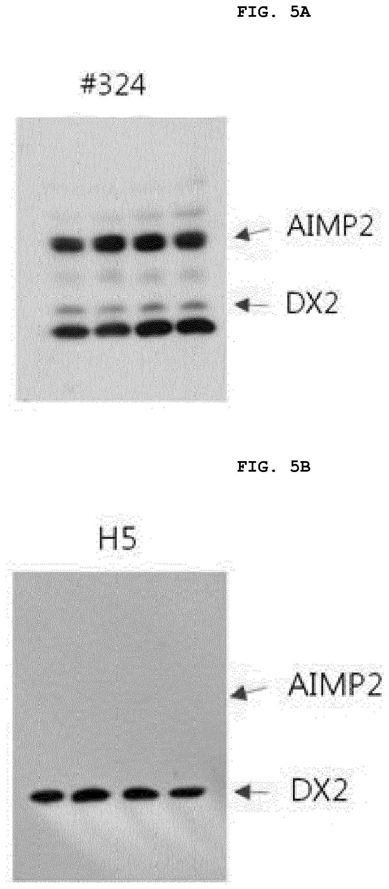

FIG. 5 shows a Western blot experiment to confirm the binding specificity of H5 Fab, it shows the result of reacting electrophoresis blot of H460 cell lysate with #324 antibody which binds both wild-type AIMP2 and AIMP2-DX2 (FIG. 5A), or H5 Fab (FIG. 5B). In the blot, the position of the protein bands of wild-type AIMP2 ("AIMP2") and AIMP2-DX2 ("DX2) are indicated by red arrows.

### Mode for Carrying Out the Invention

Hereinafter, the present invention will be described in detail with reference to the following examples.

However, the following examples are merely for illustrating the present invention and are not intended to limit the scope of the present invention.

### Example 1: Construction of chimeric Fab library

A rabbit/human chimeric Fab library was constructed for screening of antigen-binding sites that specifically bind to AIMP2-DX2. Immunization of rabbits, isolation of mRNA from rabbit splenocytes and cDNA synthesis, and construction of rabbit/human chimeric Fab antibody library are performed according to the known method in 'Phage Display: A Laboratory Manual' (Barbas, C.F. 3rd. et al. Eds. Cold Spring Harbor Laboratory Press).

Specifically, a polypeptide of the region to which exon 1 and exon 3 are conjugated in AIMP2-DX2 was used as an epitope capable of distinguishing wild-type AIMP2 protein from AIMP2-DX2 mutant protein (see Fig. 1B, amino acid sequence GHVQDYGALKD). Cysteine(C) amino acid was added to the C-terminus of the AIMP2-DX2 antigen peptide to conjugate the peptide to keyhole limpet hemocyanin(KLH), the carrier protein. KLH-conjugated AIMP2-DX2 antigen peptide was inoculated into rabbits to induce an immune response, and then mRNA was isolated from the spleens of the rabbits, and complementary DNA(cDNA) of the variable regions of rabbit antibody which is necessary for constructing chimeric Fab library was produced using reverse transcription polymerase chain reaction (RT-PCR) . The primers used for the preparation of the cDNA are as shown in Table 1. From the prepared cDNA, the DNA of the heavy chain variable region and the light chain variable region of the rabbit antibody were amplified using the combination of the primers shown in Table 2 and the PCR conditions shown in Table 3. The amplified PCR products were separated by electrophoresis in 1.0% agarose gel and purified using a gel extraction kit.

**Table 1. RT-PCR primer**

| | Forward primer | | | | | | | |
|---|---|---|---|---|---|---|---|---|
| Heavy chain (SEQ ID NO) | RVH1 (25) | RVH2 (26) | RVH3 (27) | RVH4 (28) | RVH5 (29) | RVH6 (30) | | |
| light chain(K) (SEQ ID NO) | RVK1 (31) | RVK2 (32) | RVK3 (33) | RVK4 (34) | RVK5 (35) | RVK6 (36) | RVK7 (37) | RVK8 (38) |
| light chain(λ) (SEQ ID NO) | RVL1 (39) | RVL2 (40) | RVL3 (41) | RVL4 (42) | RVL5 (43) | RVL6 (44) | | |

| | Reverse primer | | | | | | | |
|---|---|---|---|---|---|---|---|---|
| Heavy chain (SEQ ID NO) | RJH-b (45) | | | | | | | |
| light chain(K) (SEQ ID NO) | RJK1-b (46) | RJK2 -b (47) | RJK3-b (48) | RJK4-b (49) | | | | |
| light chain(λ) (SEQ ID NO) | RJL-b (50) | | | | | | | |

(The numbers in the bracket beneath the primer indicate the sequence number of the corresponding primer)

**Table 2. The combination of the primers used for RT-PCR**

| **44 combinations of the primers** | | | | | | | | |
|---|---|---|---|---|---|---|---|---|
| Heavy chain | RVH1 RJH-b | RVH2 RJH-b | RVH3 RJH-b | RVH4 RJH-b | RVH5 RJH-b | RVH6 RJH-b | | |
| light chain(κ) | RVK1 RJK1-b | RVK2 RJK1-b | RVK3 RJK1-b | RVK4 RJK1-b | RVK5 RJK1-b | RVK6 RJK1-b | RVK7 RJK1-b | RVK8 RJK1-b |
| | RVK1 RJK2-b | RVK2 RJK2-b | RVK3 RJK2-b | RVK4 RJK2-b | RVK5 RJK2-b | RVK6 RJK2-b | RVK7 RJK2-b | RVK8 RJK2-b |
| | RVK1 RJK3-b | RVK2 RJK3-b | RVK3 RJK3-b | RVK4 RJK3-b | RVK5 RJK3-b | RVK6 RJK3-b | RVK7 RJK3-b | RVK8 RJK3-b |
| | RVK1 RJK4-b | RVK2 RJK4-b | RVK3 RJK4-b | RVK4 RJK4-b | RVK5 RJK4-b | RVK6 RJK4-b | RVK7 RJK4-b | RVK8 RJK4-b |
| light chain(λ) | RVL1 RJL-b | RVL2 RJL-b | RVL3 RJL-b | RVL4 RJL-b | RVL5 RJL-b | RVL6 RJL-b | | |

**Table 3. PCR conditions for amplifying DNA of heavy chain and light chain variable region**

| PCR reaction program | 30X | | | | |
|---|---|---|---|---|---|
| 94 °C | 94 °C | 56 °C | 72 °C | 72 °C | 4°C |
| 2 min | 30 sec | 30 sec | 30 sec | 7 min | ∞ |

| | | | | | |
|---|---|---|---|---|---|
| (≈ means no time limit) | | | | | |

DNA of the constant region of human antibody (Fab) required for the construction of chimeric Fab library was obtained by PCR from pComb3XTT containing human Fab. The light and heavy chain constant regions were amplified according to the PCR conditions described in table 4 using pComb3XTT (2640.2ng/ml) plasmid as a template, and a pair of primers HKC-F (forward, SEQ ID NO: 53) and Lead-b (reverse, SEQ ID NO: 54) and a pair of primers HIgCH1-F (forward, SEQ ID NO: 51) and dpseq (reverse, SEQ ID NO: 52), respectively. The amplified PCR products were purified using gel extraction kit. The DNA sizes of the purified heavy and light chain constant regions were 400 bp and 350 bp, respectively.

**Table 4. PCR conditions for amplifying DNA of heavy chain and light chain constant region**

| PCR reaction program | 25X | | | | |
|---|---|---|---|---|---|
| 94 °C | 94 °C | 56 °C | 72 °C | 72 °C | 4°C |
| 2 min | 30 sec | 30 sec | 30 sec | 10 min | ∞ |

| | | | | | |
|---|---|---|---|---|---|
| (≈ means no time limit) | | | | | |

The DNA of the variable region of the rabbit antibody prepared above and the DNA of the constant region of the human antibody (Fab) were ligated by overlap extension PCR. The heavy chain was prepared by PCR reaction using a mixture of the heavy chain variable region DNA and the constant region DNA as a template, and primer pairs of LeadVH (SEQ ID NO: 55) and dpseq (SEQ ID NO: 52). The light chain was prepared by PCR reaction using a mixture of the light chain variable region DNA and the constant region DNA as a template, and primer pairs of RSC-SF (SEQ ID NO: 56) and Lead-b (SEQ ID NO: 54). The conditions of the PCR was as described in table 5.

**Table 5. PCR conditions for overlap extension PCR**

| PCR reaction program | 25X | | | | |
|---|---|---|---|---|---|
| 94 °C | 94 °C | 56 °C | 72 °C | 72 °C | 4°C |
| 2 min | 30 sec | 30 sec | 1 min | 10 min | ∞ |

| | | | | | |
|---|---|---|---|---|---|
| (∞ means no time limit) | | | | | |

DNA of the Fd heavy chain fragment (heavy chain constant region + heavy chain variable region) and chimeric light chain (light chain constant region + light chain variable region) prepared as described above were separated and purified using a gel extraction kit. The heavy and light chain PCR products were 750 bp and 800 bp, respectively. Finally, in order to bind the fragment of Fd heavy chain and chimeric light chain to form Fab, PCR reaction was performed according to the conditions described in Table 6 using Fd heavy chain and chimeric light chain mixture as a template, and a primer pair of RSC-SF (SEQ ID NO: 56) and dpseq (SEQ ID NO: 52). The Fab (Fd heavy chain + chimera light chain) obtained from the PCR product was electrophoresed on 1.5% gel and purified by gel extraction kit. The final product was 1500 bp in size.

**Table 6. PCR conditions for Fab construction**

| PCR reaction program | 25X | | | | |
|---|---|---|---|---|---|
| 94 °C | 94 °C | 56 °C | 72 °C | 72 °C | 4°C |
| 2 min | 30 sec | 30 sec | 1.5 min | 10 min | ∞ |

| | | | | | |
|---|---|---|---|---|---|
| (≈ means no time limit) | | | | | |

Fab DNA prepared as described above was expressed in a phage and inserted into a pComb3XTT vector so as to construct a phage-displayed Fab library. Specifically, the Fab PCR product and pComb3XTT vector were cut with SfiI restriction enzyme, and then electrophoresed on 1% gel and purified. The restriction enzyme reaction was carried out by cutting 5 µg of Fab DNA and 30 µg of pComb3XTT vector each using 2.5 µl of SfiI restriction enzyme. The sizes of the cut Fab DNA and vector are 1500 bp and 3400 bp, respectively. The digested insert DNA was mixed with the digested vector at a molar ratio of 3 : 1, reacted with T4 ligase, and then precipitated with ethanol.

The vector into which the chimeric Fab DNA was inserted was transformed into E. coli ER2537 by electroporation. SOC medium (Super Optimal Broth with Catabolite Repression-glucose added) was used for electroporation. To a cuvette kept in the cold state, 200 µl of ER2537 E. coli competent cells and the ligated vector were mixed, and after electric shock was applied, 3 ml of SOC was immediately added. The cells were then transferred to a 50 ml tube and incubated at room temperature for 1 hour, and the volumes of 1, 1/10, and 1/100 ul of the transformed cells were applied to prewarmed 100 mm LB-agar plates containing ampicillin. Separately, it was also applied to a half-divided LB-agar plate containing kanamycin and ampicillin in each half plate to check for contamination. The remnant E. coli which received electric shock was centrifuged x4,000 g at 4°C for 15 minutes, and after removing the supernatant, it was re-dispersed in 500 µl of SOC and applied to a 150 mm LB-ampicillin agar plate. The plates were incubated for about 18 hours. As a result, about 10⁷ transgenic colonies were obtained the following day.

### Example 2: Panning and Screening of phage-display Fab library

### 2-1: Panning of phage-display Fab library

The panning of the antibody (Fab) library prepared in the previous example was carried out according to a known method (Bai, X. et al. PLoS ONE 2015, doi:10.1371/journal.pone.0141045) in order to select Fabs having an antigen-binding site that specifically binds to AIMP2-DX2 antigen peptide.

As a first step of panning, a library rescue was performed in which helper phage was introduced into E. coli. Colonies grown by applying E.coli to 150 mm plate one day before the experiment, were dispersed in 5 ml of Luria-Bertani broth (LB) and collected. 100 µl of the collected bacteria were inoculated into 20ml of super broth (SB) containing ampicillin (50 µg/ml), and cultured with shaking for 3 hours. When the absorbance at 600 nm (OD 600) of the culture medium reached 0.5, 500 µl of VCSM13 helper phage (10¹² pfu) was added, and cultured with shaking at 37 °C and 120 rpm for 1 hour.

Then kanamycin (70 µg/mL) was added and cultured with shaking at 30 °C for an additional 18 hours at 220 rpm.

The culture product was centrifuged at 12,000 rpm at 4 °C for 20 minutes and the supernatant was collected and 5 ml of 5 × polyethylene glycol (PEG) precipitation solution (16% PEG-8000, 12% NaCl) was added, and the mixture was placed on ice for 30 minutes. Then, the pellet obtained by centrifugation at 12,000 rpm for 20 minutes was re-dispersed in PBS containing 1 ml of 3% BSA to prepare a phage solution, and the mixture was placed at room temperature for 1 hour. Separately, 10 ml of LB was inoculated with 160 µl of ER2537 E. coli and cultured with shaking at 37 °C, 220 rpm without antibiotics.

To an immunotube (Nunc cat. No. 470319) for panning was added 1 ml PBS containing 10 µg of AIMP2-DX2 antigen peptide (the same peptide used in the rabbit immunization, conjugated to BSA), and the tube was incubated for 1 hour at 37 °C to adsorb the antigen peptide on the inner surface of the test tube. After that, blocking was performed with PBST (PBS containing tween-20) supplemented with 3% skim milk for 1 hour, so that the surface where the antigen was not adsorbed was filled. The phage library (10¹² pfu) was blocked for 1 hour with 1 ml PBST containing 3% skim milk and 300 µg BSA.

The phage library was added to the blocked immunotube and incubated with shaking at 37 °C for 2 hours to allow binding of the Fab expressed on the phage to the antigen peptide, and washed 3 times with 5 ml of PBST. The phage bound to the immunotube was eluted with 1 ml of triethylamine (100 mM in deionized water) at room temperature for 10 minutes and immediately neutralized with 0.5 ml of Tris (1M, pH 7.0), and mixed with 8.5 ml of mid-log phase ER2537 E. coli. E. coli-phage mixture was cultured with shaking at 120 rpm and 37 °C for 1 hour, applied to LB agar plates containing 2% glucose and ampicillin, and cultured overnight at 37 °C. 10⁸ cells of E. coli grown on the plate were inoculated into 20 ml SB medium (super broth: 3% tryptone, 2% yeast extract, 1% MOPS, pH 7.0) containing ampicillin and cultured, then added 10¹² pfu of the VCSM13 helper phage when the OD600 reached 0.7. The mixture was shaken at 80 rpm, 37 °C for 1 hour and cultured overnight at 30 °C, 200 rpm with addition of kanamycin (70 µg/ml). The culture was centrifuged and the phages were precipitated with 5 ml of 5 × PEG precipitation solution (20% (w/v) PEG8000, 15% (w/v) NaCl) and left on ice for 30 minutes. The precipitated phage was centrifuged and redispersed in 0.3 ml of PBS and used for panning in the next step. The panning was repeated 4 times in the same manner as described above, and E. coli colonies selected by panning were obtained as a result.

### 2-2: Clone screening

It was confirmed that the antibodies (Fab) expressed on the phage from the selected colonies in the panning of Example <2-1> specifically bind to the AIMP2-DX2 antigen peptide.

First, among colonies which were selected by 2nd to 4th panning steps, colonies binding to BSA-conjugated AIMP2-DX2 antigen peptides were screened by ELISA according to the method of Bai, X. et al. (2015) . A 96-well immunoplate for ELISA experiments was coated with AIMP2-DX2 antigen peptide (10 µl/ml) and blocked with 180 µl of 3% BSA-PBST. E. coli culture expressing antibody (Fab) was centrifuged at 3500 rpm for 15 minutes, the cell pellets were dispersed in 40 µl of 1 × TES (20% sucrose, 1 mM EDTA, 50 mM TRIS, pH 8.0), and 60 µl of 0.2 × TES was then added to remove E.coli outer membrane and prepare periplasmic extracts. The mixtures were centrifuged and 25 µl of the supernatants were added to the immunoplate coated with the antigen peptide and allowed to react for 1 hour. The plate was washed 3 to 4 times with PBST, and 25 µl of anti-human IgG (Fab specific)-HRP (1:1000) diluted in 3% BSA-PBST was added as a secondary antibody. The plate was washed, and colorimetric reaction was performed for 10 minutes by adding 25 µl of tetramethylbenzidine (TMB) thereto. The reaction was stopped by adding 25 µl of sulfuric acid (H₂SO₄, 1 mM). The negative control of the ELISA experiment was an experiment using BSA instead of the antigen peptide, and colonies showing a signal three times stronger than the background were selected.

The binding specificity of the antibody clones selected in the ELISA for AIMP2-DX2 was confirmed using Western blot. The previously selected E. coli clones were cultured in 20 mL SB medium containing ampicillin, IPTG (1 mM final) was added when the OD600 reached 0.7, and the cells were cultured with shaking at 30 °C overnight. The E. coli culture was centrifuged and redispersed in 1 ml of cold 1 × TES, and 1.5 ml of cold 0.2 × TES was added thereto and incubated on ice for 30 minutes to prepare a periplasmic extract. The supernatant obtained by centrifugation was mixed with 3% skim milk-PBST at a ratio of 1:1 and used as a primary antibody for Western blotting. The secondary antibody was anti-HA tag antibody-HRP or anti-human Fab antibody-HRP. Western blotting using SW620 cell lysate, a colon cancer cell line expressing AIMP2-DX2, revealed that the antibody (Fab) of clone H5 specifically binds to AIMP2-DX2 of 25 kDa size (data not shown).

The sequence of the DNA encoding the Fab of the H5 clone for which the binding was confirmed by the ELISA experiment was analyzed. The DNA sequence and the amino acid sequence of the heavy chain and light chain CDR of the identified H5 Fab are set forth in SEQ ID NOS: 3 to 14 of the Sequence Listing in the present specification. In addition, the DNA sequence and the amino acid sequence of the heavy chain and light chain variable region of the H5 Fab are as shown in SEQ ID NO: 15 to SEQ ID NO: 18 in the Sequence Listing of the present specification. The base sequence and the amino acid sequence of the heavy chain region of the H5 Fab (Fd, heavy chain variable domain from rabbit antibody and heavy chain CH1 domain of human antibody) are set forth in SEQ ID NO: 24 and SEQ ID NO: 23, respectively, and the base sequence and the amino acid sequence of the light chain variable region of H5 Fab(light chain variable region derived from the rabbit antibody and the light chain Cκ Domain of the human antibody) are described in SEQ ID NO: 22 and SEQ ID NO: 21, respectively.

The H5 Fab having the unique sequence as described above was converted into the IgG type whole antibody, which is a more commonly used form. According to a conventional method of converting a Fab into an IgG antibody, the DNA of the variable region of the heavy chain and the light chain of the H5 Fab were amplified by PCR and inserted into the pVITRO1 vector in which the genes of the heavy and light chain constant regions of human IgG1 were cloned, using appropriate restriction enzymes. The DNA sequences and amino acid sequences of the heavy chain of the H5 IgG antibody produced as above (heavy chain variable region derived from rabbit antibody and heavy chain constant region of human antibody) and light chain of H5 IgG antibody (light chain variable region derived from rabbit antibody and light chain constant region Cκ domain) are as described in SEQ ID NO: 19 to SEQ ID NO: 22 in the Sequence Listing of the present specification. The DNA sequence and amino acid sequence of the light chain of the H5 IgG antibody are the same as the sequence of the light chain of the H5 Fab. The vector containing the DNA of H5 IgG was expressed in 293F cell line and separated/purified by protein G affinity chromatography.

### Example 3: Confirming the binding specificity of selected clones to AIMP2-DX2

The binding specificity of the H5 antibody or fragment (Fab) to AIMP2-DX2 was further confirmed.

First, whether H5 Fab binds specifically to AIMP2-DX2 was examined by antigen preadsorption experiment (FIG. 1). The H460 cell lysate, a lung cancer cell line expressing AIMP2-DX2, was electrophoresed, and Western blotting was performed using H5 Fab with AIMP2-DX2 antigen peptide(100 µg/ml, Fig. 1A, "+"), or without antigen peptide (Fig. 1A, "-"). The H5 Fab without the antigen peptide detected the AIMP2-DX2 protein band, but the H5 Fab with the antigen peptide did not react with the AIMP2-DX2 protein band at all and these results showed that the H5 Fab specifically binds to AIMP2-DX2.

In addition, the binding specificity of H5 Fab to AIMP2-DX2 was confirmed by inhibiting the expression of AIMP2-DX2 in H460 cells (Fig. 2). The H460 cells were transfected with the siRNA targeting exon1-exon3 junction or exon4 of AIMP2-DX2 (Fig. 2B, "siDX2" (SEQ ID NO: 57), "si exon4 #1" (SEQ ID NO: 58), "si exon4 #2" (SEQ ID NO: 59), respectively), and cultured for 72 hours. Western blotting of the cultured cell lysate with H5 Fab(FIG. 2A) revealed that the AIMP2-DX2 protein band wasn't detected with H5 Fab not only in the cell lysate transfected with siRNA targeting exon1-exon3 junction of AIMP2-DX2 but also in the cell lysate transfected with siRNA targeting exon4. It is considered that the mRNA of AIMP2-DX2 mutant was completely degraded by siRNA.

We examined whether the N-terminal amino acid deletion of AIMP2-DX2 protein affects the binding of H5 Fab to AIMP2-DX2 protein (FIG. 3). The amino acid residue at the N-terminus of the AIMP2-DX2 protein is known to have a significant effect on the solubility of the AIMP2-DX2 protein (Korean Patent Application No. 10-2014-0058634). Strep tag was ligated to AIMP2-DX2 variants in which 2, 23, or 33 amino acids were deleted at the N-terminus of AIMP2-DX2 protein (Fig. 3B) and expressed in HEK293T cells, and Western blot experiments were conducted using H5 Fab. As shown in FIG. 3A, H5 Fabs were found to bind not only to the full-length AIMP2-DX2 protein, but also to the AIMP2-DX2 isoforms with the N-terminal deletion. Meanwhile, in the presence of AIMP2-DX2 antigen peptide, H5 Fab ("H5 + epitope peptide") did not bind to either full-length or N-terminally truncated AIMP2-DX2, reaffirming that H5 Fab specifically binds to AIMP2-DX2 protein.

The binding affinity of the H5 antibody converted to IgG form to the AIMP2-DX2 antigen was confirmed by surface plasmon resonance experiment (FIG. 4). H5 antibody (IgG) and mock IgG were coated on CM5 chip, and AIMP2-DX2 antigen peptide was flowed at various concentrations and the degree of binding reaction with H5 antibody was measured. Analytical samples and buffer were injected at a flow rate of 15 µl/min for 6 min and washed for 20 min. Sensorgram was obtained by subtracting the value for the blank reaction in which protein was not coated from the reaction value for H5 antibody or mock IgG. As a result, as shown in FIG. 4, the binding of H5 antibody was increased in proportion to the concentration of the antigen peptide, but the mock IgG did not show any significant binding to the antigen peptide. It was therefore confirmed that the IgG-reformatted H5 antibody also retained the binding affinity of Fab to AIMP2-DX2 antigen.

Finally, it was determined whether the H5 antibody could differentiate between wild-type (WT) AIMP2 protein and AIMP2-DX2 (FIG. 5). Western blotting was performed with H460 cell lysate using #324 antibody which did not distinguish between wild-type AIMP2 and AIMP2-DX2, and H5 Fab, and the results were compared. In contrast to the binding of antibody #324 to both wild-type AIMP2 and AIMP2-DX2 (Fig. 5A), H5 Fab did not bind to wild-type AIMP2 at all but specifically bound to AIMP2-DX2 (Fig. 5B). In other words, it was confirmed that only AIMP2-DX2 could be selectively detected using H5 antibody. Since the H5 IgG antibody contains the variable regions of the H5 Fab without modification, and retains the binding characteristics to the antigen, it can be seen that the H5 IgG antibody can give the same result as the H5 Fab.

### Industrial Applicability

As described above, since the antibody according to the present invention doesn't bind to wild-type AIMP2, but specifically binds to AIMP2-DX2 mutant protein which has exon2 deletion, it can be usefully utilized to confirm the presence and expression level of AIMP2-DX2 protein. AIMP2-DX2 protein is associated with pathologies such as cancer and inflammatory diseases and is overexpressed in cancers such as lung cancer, breast cancer, colorectal cancer and the like. Therefore, it can be usefully utilized to develop a composition for diagnosing a cancer by detecting AIMP2-DX2 as a biomarker for cancer development and progression.

## Claims

1. An antibody or fragment thereof that specifically binds to AIMP2-DX2, the antibody or fragment thereof recognizing a polypeptide comprising the amino acid sequence of SEQ ID NO: 1 as an epitope.

2. The antibody or fragment thereof of claim 1, wherein the antibody or fragment thereof comprises:
a heavy chain variable region comprising a heavy chain complementary-determining region (CDR) 1 comprising the amino acid sequence of SEQ ID NO: 3, a heavy chain CDR2 comprising the amino acid sequence of SEQ ID NO: 5 and a heavy chain CDR3 comprising the amino acid sequence of SEQ ID NO: 7; and
a light chain variable region comprising a light chain CDR1 comprising the amino acid sequence of SEQ ID NO: 9, a light chain CDR2 comprising the amino acid sequence of SEQ ID NO: 11 and a light chain CDR3 comprising the amino acid sequence of SEQ ID NO: 13.

3. The antibody or fragment thereof of claim 1, wherein the antibody or fragment thereof comprises a heavy chain variable region comprising the amino acid sequence of SEQ ID NO: 15; and a light chain variable region comprising the amino acid sequence of SEQ ID NO: 17.

4. The antibody or fragment thereof of claim 1, wherein the antibody comprises a heavy chain comprising the amino acid sequence of SEQ ID NO: 19; and a light chain comprising the amino acid sequence of SEQ ID NO: 21.

5. The antibody or fragment thereof of claim 1, wherein the antibody is selected from the group consisting of IgG, IgA, IgM, IgE and IgD.

6. The antibody or fragment thereof of claim 1, wherein the fragment is selected from the group consisting of diabody, Fab, Fab', F(ab)₂, F(ab')₂, Fv, and scFv fragments.

7. A composition for diagnosing a cancer comprising the antibody or fragment thereof of any one of claims 1 to 6.

8. The composition of claim 7, wherein the cancer is selected from the group consisting of colorectal cancer, colon cancer, lung cancer, liver cancer, gastric cancer, esophageal cancer, pancreatic cancer, gallbladder cancer, kidney cancer, bladder cancer, prostate cancer, testicular cancer, cervical cancer, endometrial cancer, villous cancer, ovarian cancer, breast cancer, thyroid cancer, brain cancer, head and neck cancer, melanoma, lymphoma, aplastic anemia and blood cancer.

9. A polynucleotide encoding the antibody or fragment thereof of any one of claims 1 to 6.

10. The polynucleotide of claim 9, wherein the polynucleotide comprises the nucleic acid sequence of SEQ ID NO: 16 or 18.

11. A recombinant expression vector comprising the polynucleotide of claim 10.

12. A cell transformed with the recombinant expression vector of claim 11.

13. A method for preparing an antibody or fragment thereof that specifically binds to AIMP2-DX2, the method comprising the steps of:
(a) transforming a host cell with the recombinant expression vector of claim 12;
(b) culturing the host cell and producing the antibody or fragment thereof; and
(c) obtaining the antibody or fragment thereof produced in the host cell.

14. Use of the antibody or fragment thereof of any one of claims 1 to 6 for preparing an agent for diagnosing a cancer.

15. A method for diagnosing a cancer in a subject, the method comprising the steps of:
(a) obtaining a biological sample from the subject;
(b) determining the level of AIMP2-DX2 protein in the biological sample using the antibody or fragment thereof of any one of claims 1 to 6; and
(c) comparing the determined level of AIMP2-DX2 protein with the level of AIMP2-DX2 protein in a normal subject.


<110>    Medicinal Bioconvergence Research Center
         Ewha University - Industry Collaboration Foundation

<120>    ANTIBODY SPECIFICALLY BINDING TO AIMP2-DX2 PROTEIN

<130>    OP18-0113/PCT/EP

<150>    KR 10-2016-0028809
<151>    2016-03-10

<150>    PCT/KR 2017/002631
<151>    2017-03-10

<160>    59

<170>    KopatentIn 2.0

<210>    1
<211>    11
<212>    PRT
<213>    Artificial Sequence

<220>
<223>    AIMP2-DX2 antigen peptide


<400>    1
Gly His Val Gln Asp Tyr Gly Ala Leu Lys Asp
  1               5                  10


<210>    2
<211>    33
<212>    DNA
<213>    Artificial Sequence

<220>
<223>    AIMP2-DX2 antigen peptide


<400>    2
ggccacgtgc aggattacgg ggcgctgaaa gac                                    33


<210>    3
<211>    5
<212>    PRT
<213>    Artificial Sequence

<220>
<223>    H5 heavy chain CDR1


<400>    3
Ser Tyr His Met Thr
  1               5


<210>    4
<211>    15
<212>    DNA
<213>    Artificial Sequence

<220>
<223>    H5 heavy chain CDR1

<400>    4
agctaccaca tgacc                                                        15

<210>    5
<211>    16
<212>    PRT
<213>    Artificial Sequence

<220>
<223>    H5 heavy chain CDR2

<400>    5
Val Ile Ser Asn Ser Gly Gly Thr Ser Tyr Ala Asn Trp Ala Lys Gly
 1               5                  10                  15

<210>    6
<211>    48
<212>    DNA
<213>    Artificial Sequence

<220>
<223>    H5 heavy chain CDR2

<400>    6
gtcattagta atagtggtgg cacatcctac gcgaactggg cgaaaggc                    48

<210>    7
<211>    11
<212>    PRT
<213>    Artificial Sequence

<220>
<223>    H5 heavy chain CDR3

<400>    7
Val Arg Gly Val Pro Gly Ile Asn Ser Asn Leu
 1               5                  10

<210>    8
<211>    33
<212>    DNA
<213>    Artificial Sequence

<220>
<223>    H5 heavy chain CDR3

<400>    8
gtcagagggg tgcctggtat taatagtaac ttg                                    33

<210>    9
<211>    13
<212>    PRT
<213>    Artificial Sequence

<220>
<223>    H5 light chain CDR1


<400>    9
Gln Ser Ser Gln Ser Val Tyr Ser Gly Asn Trp Leu Ala
1               5                   10


<210>    10
<211>    39
<212>    DNA
<213>    Artificial Sequence

<220>
<223>    H5 light chain CDR1


<400>    10
cagtccagtc agagtgttta tagtggcaac tggttagcc                                39


<210>    11
<211>    7
<212>    PRT
<213>    Artificial Sequence

<220>
<223>    H5 light chain CDR2


<400>    11
Ser Thr Ser Thr Leu Ala Ser
1               5


<210>    12
<211>    21
<212>    DNA
<213>    Artificial Sequence

<220>
<223>    H5 light chain CDR2


<400>    12
tctacatcca ctctggcatc t                                                   21


<210>    13
<211>    10
<212>    PRT
<213>    Artificial Sequence

<220>
<223>    H5 light chain CDR3

<400>    13
Ala Gly Asp Tyr Asp Gly Tyr Lys Asn Ala
1               5                   10


<210>    14
<211>    30
<212>    DNA
<213>    Artificial Sequence

<220>
<223>    H5 light chain CDR3


<400>    14
gcaggcgatt atgatggtta caaaaatgct                                        30


<210>    15
<211>    115
<212>    PRT
<213>    Artificial Sequence

<220>
<223>    H5 heavy chain variable domain (VH)


<400>    15
Gln Glu Gln Leu Val Glu Ser Gly Gly Gly Leu Val Thr Pro Gly Gly
1               5                   10                  15

Thr Leu Thr Leu Thr Cys Thr Ala Ser Gly Phe Thr Ile Ser Ser Tyr
            20                  25                  30

His Met Thr Trp Val Arg Gln Ala Pro Gly Lys Gly Leu Glu Trp Ile
        35                  40                  45

Gly Val Ile Ser Asn Ser Gly Gly Thr Ser Tyr Ala Asn Trp Ala Lys
    50                  55                  60

Gly Arg Phe Thr Ile Ser Lys Thr Ser Thr Thr Val Asp Leu Lys Ile
65                  70                  75                  80

Thr Ser Pro Thr Thr Glu Asp Thr Ala Thr Tyr Phe Cys Val Arg Gly
                85                  90                  95

Val Pro Gly Ile Asn Ser Asn Leu Trp Gly Gln Gly Thr Leu Val Thr
            100                 105                 110

Ile Ser Ser
        115


<210>    16
<211>    345
<212>    DNA
<213>    Artificial Sequence

<220>
<223>    H5 heavy chain variable domain (VH)


<400>    16

caggagcagc tggtggagtc cggaggaggc ctggtcacgc ctggaggaac cctgacactc 60

acctgcacag cctctggatt caccatcagt agctaccaca tgacctgggt ccgccaggct 120

ccagggaagg ggctggaatg gatcggagtc attagtaata gtggtggcac atcctacgcg 180

aactgggcga aaggccgatt caccatctcc aaaacctcga ccacggtgga cctgaaaatc 240

accagtccga caaccgagga cacggccacc tatttctgtg tcagaggggt gcctggtatt 300

aatagtaact tgtggggcca aggcaccctg gtcaccatct cctca 345

<210> 17
<211> 110
<212> PRT
<213> Artificial Sequence

<220>
<223> H5 light chain variable domain (VL)

<400> 17
Ala Gln Val Leu Thr Gln Thr Pro Ser Ser Val Ser Ala Ala Val Gly
1               5                   10                  15

Gly Thr Val Thr Ile Asn Cys Gln Ser Ser Gln Ser Val Tyr Ser Gly
            20                  25                  30

Asn Trp Leu Ala Trp Tyr Gln Gln Lys Pro Gly Gln Pro Pro Lys Leu
        35                  40                  45

Leu Ile Tyr Ser Thr Ser Thr Leu Ala Ser Gly Val Pro Ser Arg Phe
    50                  55                  60

Lys Gly Ser Gly Ser Gly Thr Gln Phe Thr Leu Thr Val Ser Asp Leu
65                  70                  75                  80

Glu Cys Asp Asp Ala Ala Thr Tyr Tyr Cys Ala Gly Asp Tyr Asp Gly
                85                  90                  95

Tyr Lys Asn Ala Phe Gly Gly Gly Thr Lys Val Val Val Lys
            100                 105                 110

<210> 18
<211> 330
<212> DNA
<213> Artificial Sequence

<220>
<223> H5 light chain variable domain (VL)

<400> 18
gctcaagtgc tgacccagac tccatcctcc gtgtctgcag ctgtgggagg cacagtcacc 60

atcaattgcc agtccagtca gagtgtttat agtggcaact ggttagcctg gtatcagcag 120

aaaccagggc agcctcccaa gctcctgatc tattctacat ccactctggc atctggggtc 180

ccatcgcggt tcaaaggcag tggatctggg acacagttca ctctcaccgt cagcgacctg 240

gagtgtgacg atgctgccac ttactactgt gcaggcgatt atgatggtta caaaaatgct 300

ttcggcggag ggaccaaggt ggtcgtcaaa 330

<210> 19
<211> 445
<212> PRT
<213> Artificial Sequence

<220>
<223> H5 IgG heavy chain (HC)

<400> 19
Gln Glu Gln Leu Val Glu Ser Gly Gly Gly Leu Val Thr Pro Gly Gly
1               5                   10                  15

Thr Leu Thr Leu Thr Cys Thr Ala Ser Gly Phe Thr Ile Ser Ser Tyr
            20                  25                  30

His Met Thr Trp Val Arg Gln Ala Pro Gly Lys Gly Leu Glu Trp Ile
        35                  40                  45

Gly Val Ile Ser Asn Ser Gly Gly Thr Ser Tyr Ala Asn Trp Ala Lys
    50                  55                  60

Gly Arg Phe Thr Ile Ser Lys Thr Ser Thr Thr Val Asp Leu Lys Ile
65                  70                  75                  80

Thr Ser Pro Thr Thr Glu Asp Thr Ala Thr Tyr Phe Cys Val Arg Gly
                85                  90                  95

Val Pro Gly Ile Asn Ser Asn Leu Trp Gly Gln Gly Thr Leu Val Thr
            100                 105                 110

Ile Ser Ser Ala Ser Thr Lys Gly Pro Ser Val Phe Pro Leu Ala Pro
        115                 120                 125

Ser Ser Lys Ser Thr Ser Gly Gly Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly
            180                 185                 190

Thr Gln Thr Tyr Ile Cys Asn Val Asn His Lys Pro Ser Asn Thr Lys
        195                 200                 205

Val Asp Lys Lys Val Glu Pro Lys Ser Cys Asp Lys Thr His Thr Cys
    210                 215                 220

Pro Pro Cys Pro Ala Pro Glu Leu Leu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Lys
            260                 265                 270

Phe Asn Trp Tyr Val Asp Gly Val Glu Val His Asn Ala Lys Thr Lys
        275                 280                 285

Pro Arg Glu Glu Gln Tyr Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Ala Leu Pro Ala Pro Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Arg Asp Glu Leu Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Gln Gly Asn Val Phe Ser Cys Ser Val Met His Glu Ala Leu His Asn
            420                 425                 430

His Tyr Thr Gln Lys Ser Leu Ser Leu Ser Pro Gly Lys
        435                 440                 445


<210>    20
<211>    1335
<212>    DNA
<213>    Artificial Sequence

<220>
<223>    H5 IgG heavy chain (HC)


<400>    20
caggagcagc tggtggagtc cggaggaggc ctggtcacgc ctggaggaac cctgacactc      60

acctgcacag cctctggatt caccatcagt agctaccaca tgacctgggt ccgccaggct     120

ccagggaagg ggctggaatg gatcggagtc attagtaata gtggtggcac atcctacgcg     180

aactgggcga aaggccgatt caccatctcc aaaacctcga ccacggtgga cctgaaaatc     240

accagtccga caaccgagga cacggccacc tatttctgtg tcagaggggt gcctggtatt     300

aatagtaact tgtggggcca aggcaccctg gtcaccatct cctcagcctc caccaagggc     360

ccatcggtct tccccctggc accctcctcc aagagcacct ctgggggcac agcggccctg     420

ggctgcctgg tcaaggacta cttccccgaa ccggtgacgg tgtcgtggaa ctcaggcgcc     480

ctgaccagcg gcgtgcacac cttcccggct gtcctacagt cctcaggact ctactccctc 540

agcagcgtgg tgaccgtgcc ctccagcagc ttgggcaccc agacctacat ctgcaacgtg 600

aatcacaagc ccagcaacac caaggtggac aagaaagttg agcccaaatc ttgtgacaaa 660

actcacacat gcccaccgtg cccagcacct gaactcctgg ggggaccgtc agtcttcctc 720

ttccccccaa aacccaagga caccctcatg atctcccgga cccctgaggt cacatgcgtg 780

gtggtggacg tgagccacga agaccctgag gtcaagttca actggtacgt ggacggcgtg 840

gaggtgcata atgccaagac aaagccgcgg gaggagcagt acaacagcac gtaccgtgtg 900

gtcagcgtcc tcaccgtcct gcaccaggac tggctgaatg gcaaggagta caagtgcaag 960

gtctccaaca aagccctccc agcccccatc gagaaaacca tctccaaagc caaagggcag 1020

ccccgagaac cacaggtgta caccctgccc ccatcccggg atgagctgac caagaaccag 1080

gtcagcctga cctgcctggt caaaggcttc tatcccagcg acatcgccgt ggagtgggag 1140

agcaatgggc agccggagaa caactacaag accacgcctc ccgtgctgga ctccgacggc 1200

tccttcttcc tctacagcaa gctcaccgtg gacaagagca ggtggcagca ggggaacgtc 1260

ttctcatgct ccgtgatgca tgaggctctg cacaaccact acacgcagaa gagcctctcc 1320

ctgtccccgg gtaaa 1335

<210> 21
<211> 217
<212> PRT
<213> Artificial Sequence

<220>
<223> H5 IgG light chain (LC)

<400> 21
Ala Gln Val Leu Thr Gln Thr Pro Ser Ser Val Ser Ala Ala Val Gly
1               5                   10                  15

Gly Thr Val Thr Ile Asn Cys Gln Ser Ser Gln Ser Val Tyr Ser Gly
            20                  25                  30

Asn Trp Leu Ala Trp Tyr Gln Gln Lys Pro Gly Gln Pro Pro Lys Leu
        35                  40                  45

Leu Ile Tyr Ser Thr Ser Thr Leu Ala Ser Gly Val Pro Ser Arg Phe
    50                  55                  60

Lys Gly Ser Gly Ser Gly Thr Gln Phe Thr Leu Thr Val Ser Asp Leu
65                  70                  75                  80

Glu Cys Asp Asp Ala Ala Thr Tyr Tyr Cys Ala Gly Asp Tyr Asp Gly
                85                  90                  95

Tyr Lys Asn Ala Phe Gly Gly Gly Thr Lys Val Val Val Lys Arg Thr
            100                 105                 110

Val Ala Ala Pro Ser Val Phe Ile Phe Pro Pro Ser Asp Glu Gln Leu

115                 120                 125

Lys Ser Gly Thr Ala Ser Val Val Cys Leu Leu Asn Asn Phe Tyr Pro
    130                 135                 140

Arg Glu Ala Lys Val Gln Trp Lys Val Asp Asn Ala Leu Gln Ser Gly
145                 150                 155                 160

Asn Ser Gln Glu Ser Val Thr Glu Gln Asp Ser Lys Asp Ser Thr Tyr
                165                 170                 175

Ser Leu Ser Ser Thr Leu Thr Leu Gly Lys Ala Asp Tyr Glu Lys His
            180                 185                 190

Lys Val Tyr Ala Cys Glu Val Thr His Gln Gly Leu Ser Leu Pro Val
        195                 200                 205

Thr Lys Ser Phe Asn Arg Gly Glu Cys
    210                 215


<210>    22
<211>    651
<212>    DNA
<213>    Artificial Sequence

<220>
<223>    H5 IgG light chain (LC)


<400>    22
gctcaagtgc tgacccagac tccatcctcc gtgtctgcag ctgtgggagg cacagtcacc      60

atcaattgcc agtccagtca gagtgtttat agtggcaact ggttagcctg gtatcagcag     120

aaaccagggc agcctcccaa gctcctgatc tattctacat ccactctggc atctggggtc     180

ccatcgcggt tcaaaggcag tggatctggg acacagttca ctctcaccgt cagcgacctg     240

gagtgtgacg atgctgccac ttactactgt gcaggcgatt atgatggtta caaaaatgct     300

ttcggcggag ggaccaaggt ggtcgtcaaa cgaactgtgg ctgcaccatc tgtcttcatc     360

ttcccgccat ctgatgagca gttgaaatct ggaactgcct ctgttgtgtg cctgctgaat     420

aacttctatc ccagagaggc caaagtacag tggaaggtgg ataacgccct ccaatcgggt     480

aactcccagg agagtgtcac agagcaggac agcaaggaca gcacctacag cctcagcagc     540

accctgacgc tgggcaaagc agactacgag aaacacaaag tctacgcctg cgaagtcacc     600

catcagggcc tgagcttgcc cgtcacaaag agcttcaaca ggggagagtg t              651


<210>    23
<211>    221
<212>    PRT
<213>    Artificial Sequence

<220>
<223>    H5 Fd heavy chain (heavy chain portion of H5 Fab)

<400>    23
Gln Glu Gln Leu Val Glu Ser Gly Gly Gly Leu Val Thr Pro Gly Gly
1               5                   10                  15

Thr Leu Thr Leu Thr Cys Thr Ala Ser Gly Phe Thr Ile Ser Ser Tyr
            20                  25                  30

His Met Thr Trp Val Arg Gln Ala Pro Gly Lys Gly Leu Glu Trp Ile
        35                  40                  45

Gly Val Ile Ser Asn Ser Gly Gly Thr Ser Tyr Ala Asn Trp Ala Lys
    50                  55                  60

Gly Arg Phe Thr Ile Ser Lys Thr Ser Thr Thr Val Asp Leu Lys Ile
65                  70                  75                  80

Thr Ser Pro Thr Thr Glu Asp Thr Ala Thr Tyr Phe Cys Val Arg Gly
                85                  90                  95

Val Pro Gly Ile Asn Ser Asn Leu Trp Gly Gln Gly Thr Leu Val Thr
            100                 105                 110

Ile Ser Ser Ala Ser Thr Lys Gly Pro Ser Val Phe Pro Leu Ala Pro
        115                 120                 125

Ser Ser Lys Ser Thr Ser Gly Gly Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly
            180                 185                 190

Thr Gln Thr Tyr Ile Cys Asn Val Asn His Lys Pro Ser Asn Thr Lys
        195                 200                 205

Val Asp Lys Lys Val Glu Pro Lys Ser Cys Asp Lys Thr
    210                 215                 220

<210>    24
<211>    663
<212>    DNA
<213>    Artificial Sequence

<220>
<223>    H5 Fd heavy chain (heavy chain portion of H5 Fab)

<400>    24
caggagcagc tggtggagtc cggaggaggc ctggtcacgc ctggaggaac cctgacactc      60

acctgcacag cctctggatt caccatcagt agctaccaca tgacctgggt ccgccaggct     120

ccagggaagg ggctggaatg gatcggagtc attagtaata gtggtggcac atcctacgcg     180

aactgggcga aaggccgatt caccatctcc aaaacctcga ccacggtgga cctgaaaatc     240

accagtccga caaccgagga cacggccacc tatttctgtg tcagaggggt gcctggtatt 300

aatagtaact tgtggggcca aggcaccctg gtcaccatct cctcagcctc caccaagggc 360

ccatcggtct tccccctggc accctcctcc aagagcacct ctgggggcac agcggccctg 420

ggctgcctgg tcaaggacta cttccccgaa ccggtgacgg tgtcgtggaa ctcaggcgcc 480

ctgaccagcg gcgtgcacac cttcccggct gtcctacagt cctcaggact ctactccctc 540

agcagcgtgg tgaccgtgcc ctccagcagc ttgggcaccc agacctacat ctgcaacgtg 600

aatcacaagc ccagcaacac caaggtggac aagaaagttg agcccaaatc ttgtgacaaa 660

act 663

<210> 25
<211> 35
<212> DNA
<213> Artificial Sequence

<220>
<223> RVH1 primer

<400> 25
gcccaaccag ccatggccca ggagcagcta aggag 35

<210> 26
<211> 36
<212> DNA
<213> Artificial Sequence

<220>
<223> RVH2 primer

<400> 26
gcccaaccag ccatggccca ggagcagctg rtggag 36

<210> 27
<211> 39
<212> DNA
<213> Artificial Sequence

<220>
<223> RVH3 primer

<400> 27
gcccaaccag ccatggccca ggagcagctg gaggagtcc 39

<210> 28
<211> 36
<212> DNA
<213> Artificial Sequence

<220>
<223> RVH4 primer

<400>    28
gcccaaccag ccatggccca gtcgstggag gagtcc                                36


<210>    29
<211>    36
<212>    DNA
<213>    Artificial Sequence

<220>
<223>    RVH5 primer


<400>    29
gcccaaccag ccatggccca gtcggtgaag gagtcc                                36


<210>    30
<211>    36
<212>    DNA
<213>    Artificial Sequence

<220>
<223>    RVH6 primer


<400>    30
gcccaaccag ccatggccca gcagctggag cagtcc                                36


<210>    31
<211>    36
<212>    DNA
<213>    Artificial Sequence

<220>
<223>    RVK1 primer


<400>    31
taattggccc aggcggccga ccctatgctg acccag                                36


<210>    32
<211>    36
<212>    DNA
<213>    Artificial Sequence

<220>
<223>    RVK2 primer


<400>    32
taattggccc aggcggccga tgtcgtgatg acccag                                36


<210>    33
<211>    36
<212>    DNA
<213>    Artificial Sequence

<220>
<223>    RVK3 primer


<400>    33
taattggccc aggcggccgc agccgtgctg acccag                                36


<210>    34
<211>    36
<212>    DNA
<213>    Artificial Sequence

<220>
<223>    RVK4 primer


<400>    34
taattggccc aggcggccgc catcgatatg acccag                                36


<210>    35
<211>    36
<212>    DNA
<213>    Artificial Sequence

<220>
<223>    RVK5 primer


<400>    35
taattggccc aggcggccgc ccaagtgctg acccag                                36


<210>    36
<211>    36
<212>    DNA
<213>    Artificial Sequence

<220>
<223>    RVK6 primer


<400>    36
taattggccc aggcggccgc ccttgtgatg acccag                                36


<210>    37
<211>    36
<212>    DNA
<213>    Artificial Sequence

<220>
<223>    RVK7 primer


<400>    37
taattggccc aggcggccgc tcaagtgctg acccag                                36


<210>    38
<211>    36
<212>    DNA

<213>    Artificial Sequence

<220>
<223>    RVK8 primer


<400>    38
taattggccc aggcggccta tgtcatgatg acccag                                36


<210>    39
<211>    36
<212>    DNA
<213>    Artificial Sequence

<220>
<223>    RVL1 primer


<400>    39
taattggccc aggcggccca gcctgccctc actcag                                36


<210>    40
<211>    36
<212>    DNA
<213>    Artificial Sequence

<220>
<223>    RVL2 primer


<400>    40
taattggccc aggcggcctc ctatgagctg acacag                                36


<210>    41
<211>    36
<212>    DNA
<213>    Artificial Sequence

<220>
<223>    RVL3 primer


<400>    41
taattggccc aggcggcctc cttcgtgctg actcag                                36


<210>    42
<211>    36
<212>    DNA
<213>    Artificial Sequence

<220>
<223>    RVL4 primer


<400>    42
taattggccc aggcggccca gcctgtgctg actcag                                36


<210>    43

<211>    36
<212>    DNA
<213>    Artificial Sequence

<220>
<223>    RVL5 primer


<400>    43
taattggccc aggcggccag cgttgtgttc acgcag                                36


<210>    44
<211>    36
<212>    DNA
<213>    Artificial Sequence

<220>
<223>    RVL6 primer


<400>    44
taattggccc aggcggccca gtttgtgctg actcag                                36


<210>    45
<211>    39
<212>    DNA
<213>    Artificial Sequence

<220>
<223>    RJH-b primer


<400>    45
tgggcccttg gtggaggctg argagayggt gaccagggt                             39


<210>    46
<211>    39
<212>    DNA
<213>    Artificial Sequence

<220>
<223>    RJK1-b primer


<400>    46
agatggtgca gccacagttc gtttgatttc cacattggt                             39


<210>    47
<211>    39
<212>    DNA
<213>    Artificial Sequence

<220>
<223>    RJK2-b primer


<400>    47
agatggtgca gccacagttc gttygacsac cacctyggt                             39

<210>    48
<211>    39
<212>    DNA
<213>    Artificial Sequence

<220>
<223>    RJK3-b primer


<400>    48
agatggtgca gccacagttc gtaggatctc cagctcggt                                39


<210>    49
<211>    39
<212>    DNA
<213>    Artificial Sequence

<220>
<223>    RJK4-b primer


<400>    49
agatggtgca gccacagttc gtttgatytc cascttggt                                39


<210>    50
<211>    42
<212>    DNA
<213>    Artificial Sequence

<220>
<223>    RJL-b primer


<400>    50
agatggtgca gccacagttc ggcctgtgac ggtcagctgg gt                            42


<210>    51
<211>    18
<212>    DNA
<213>    Artificial Sequence

<220>
<223>    HIgCH1-f primer


<400>    51
gcctccacca agggccca                                                       18


<210>    52
<211>    19
<212>    DNA
<213>    Artificial Sequence

<220>
<223>    dpseq primer


<400>    52

agaagcgtag tccggaacg                                                    19

<210>    53
<211>    19
<212>    DNA
<213>    Artificial Sequence

<220>
<223>    HKC-f primer

<400>    53
actgtggctg caccatctg                                                    19

<210>    54
<211>    18
<212>    DNA
<213>    Artificial Sequence

<220>
<223>    Lead-b primer

<400>    54
ggccatggct ggttgggc                                                     18

<210>    55
<211>    18
<212>    DNA
<213>    Artificial Sequence

<220>
<223>    LeadVH primer

<400>    55
gcccaaccag ccatggcc                                                     18

<210>    56
<211>    18
<212>    DNA
<213>    Artificial Sequence

<220>
<223>    RSC-SF primer

<400>    56
taattggccc aggcggcc                                                     18

<210>    57
<211>    19
<212>    DNA
<213>    Artificial Sequence

<220>
<223>    siDX2 siRNA

<400>    57
ctggccacgt gcaggatta                                                    19


<210>    58
<211>    19
<212>    DNA
<213>    Artificial Sequence

<220>
<223>    si exon4 #1 siRNA


<400>    58
ggaacattgc acgtttctt                                                    19


<210>    59
<211>    19
<212>    DNA
<213>    Artificial Sequence

<220>
<223>    si exon4 #2 siRNA


<400>    59
gctgtcaacg caaccctta                                                    19